Europäisches Patentamt

European Patent Office

Office européen des brevets

(11) Publication number: **0 444 742 A1**

# EUROPEAN PATENT APPLICATION

(21) Application number: 91200380.3

(22) Date of filing: **21.02.91**

(51) Int. Cl.⁵: **C07C 69/145**, C07C 67/11,
C07C 33/025, C07C 27/02,
//C07C21/14

(30) Priority: **26.02.90 GB 9004282**

(43) Date of publication of application:
**04.09.91 Bulletin 91/36**

(84) Designated Contracting States:
**AT BE CH DE FR GB IT LI NL SE**

(71) Applicant: **SHELL INTERNATIONALE
RESEARCH MAATSCHAPPIJ B.V.
Carel van Bylandtlaan 30
NL-2596 HR Den Haag(NL)**

(72) Inventor: **Kramer, Petrus Anthonius
Badhuisweg 3
NL-1031 CM Amsterdam(NL)**
Inventor: **Wullink-Schelvis, Anette Maria
Badhuisweg 3
NL-1031 CM Amsterdam(NL)**

(74) Representative: **Tuijn, Jan Warnaar et al
Shell Internationale Research Maatschappij
B.V., Patents, Licensing & Trade Marks
Division, P.O. Box 302
NL-2501 CH The Hague(NL)**

(54) Ester/alcohol synthesis.

(57) A process for preparing an ester of the formula

$R^1\text{-O-CO-}R^2$

wherein $R^1$ is an alkenyl group and $R^2$ is an alkyl, alkenyl or alkynyl group, which comprises reacting the corresponding halide of formula $R^1$Hal with the corresponding alkali metal acylate of the formula $MOCOR^2$, in the presence of an inert aprotic solvent and a phase-transfer catalyst, optionally followed by hydrolysis of the ester into the corresponding alcohol.

EP 0 444 742 A1

This invention relates to a process for preparing esters, and thereby the corresponding alcohols, by reacting a halide with a metal acylate and, if desired, hydrolysis of the ester into the corresponding alcohol.

Omega-Decenol (9-decen-1-ol; hereinafter "DOL") is a valuable synthon. EP-A-0187702 describes the use of DOL in pharmaceutical film preparation. Omega-Decenyl acetate (dec-9-enyl acetate; hereinafter "DAC") is also of interest, as a building block for prostanoid synthons (see C.S. Subramaniam, Tetrahedron Lett., (5), 495-6). Further, DOL finds use as an aroma chemical.

The acetoxylation of halo-alkyl, -alkenyl and -alkynyl compounds is known. For example, the acetoxylation reaction has been claimed for the preparation of 7-acetoxyhept-2-yn-1-ol from 7-chlorohept-2-yn-1-ol and 1,4-diacetoxy-2-butene from 1,4-dibromo-2-butene using potassium acetate and polyethylene glycol 400 (DD-A-0214374) and acetic acid/acetic anhydride (US-A-4297506) as solvent respectively. Similar acetoxylation reactions using sodium acetate are disclosed in JP 60/25954. For large-scale production, however, the operability of such processes is poor, at least for the acetoxylation of an omega-decenyl bromide.

Currently, omega-decenol is manufactured by catalysed dehydration of 1,10-decanediol at high temperature (300-500 $^{\circ}$C). This results generally in incomplete conversion, and moderate selectivity, reportedly 95% and 85% respectively. This and other known syntheses of DOL are as follows:

DE-A-3510568: Dehydration of alpha-omega diols at 300-500 $^{\circ}$C over alkaline earth metal catalysts.

DE-A-2904518: Dehydration of alpha-omega diols over pyrophosphate salts at 410 $^{\circ}$C.

Normant, Tetrahedron Lett. (1985): Preparation of a protected (ethoxyethyl ether) DOL using organo-copper reagents to couple an acetylene to $RO-(CH_2)_8-Li$.

DE-A-3331929: Thermal decomposition of polycarbonates in the presence of a Ti catalyst: 1,10-decanediol reacted with diethyl carbonate at 140 $^{\circ}$C., contacted with Ti acetylacetonate and heated to 225-310 $^{\circ}$C gave 94% conversion and 76% selectivity to DOL.

Tagasago: Bull. Chem. Soc. Japan (1981): Dehydration of alpha-omega diols at 330-50 $^{\circ}$C using stearic/palmitic acid to give DOL with ca. 70% selectivity.

Bhabha, Tet. Lett. (1978): Grignard coupling of alpha-omega alkane bromo ether with $CH2 = CH-(CH2)n-MgBr$.

JP-A-51125204: Metathesis reaction of alpha-omega halo olefins with olefins to provide e.g. omega-decen-1-yl chloride fraction which is treated with AcOH/DMF to give alpha-omega olefin acetate which is then hydrolysed.

Erlangen, Chem. Ber. (1975): Wittig reaction.

It has been found that acetic acid is not a satisfactory acetoxylating agent for the preparation of DAC from 10-bromo-1-decene (hereinafter "BDE").

According to the present invention, a process for preparing an ester of the formula

$R^1-O-CO-R^2$

wherein $R^1$ is an alkenyl group and $R^2$ is an alkyl, alkenyl or alkynyl group, comprises reacting the corresponding halide of formula $R^1Hal$ with the corresponding alkali metal acylate of formula $MOCOR^2$, in the presence of an inert aprotic solvent and a phase-transfer catalyst.

The acylation, e.g. acetoxylation reaction of the invention allows the ready removal of inorganic components. Water or another solvent is added to dissolve metal halides MHal precipitated during the reaction and also serves to remove excess/unreacted $MOCOR^2$ from the reaction mixture. The two phases so formed are allowed to separate, and the (lower) Hal layer can be run off or otherwise separated. Alternatively, the precipitate may be removed by filtration. The aprotic solvent can be removed from the ester, e.g. by vacuum flash-evaporation.

The reaction may be effected in the presence of any suitable inert aprotic solvent, for example alkanes and aromatic solvents such as toluene and xylene. Toluene and xylene are preferred solvents.

The reaction may be conveniently effected at a temperature between ambient temperature and the temperature at which the phase-transfer catalyst decomposes, typically at a temperature between ambient temperature and about 140 $^{\circ}$C. The pressure under which the reaction may be effected is dependant upon the temperature of the reaction mixture and the boiling point of the solvent being used. The use of toluene or xylene as solvent advantageously allows the reaction to be effected at a temperature of up to 110 $^{\circ}$C in the case of toluene and up to 140 $^{\circ}$C in the case of xylene, both at ambient pressure.

The novel process is of general applicability. Accordingly, $R^1$ is an alkenyl group and $R^2$ may be selected from alkyl, alkenyl or alkynyl groups, the groups optionally bearing one or more substituents, for example aromatic groups, such as phenyl, aromatic heterocyclic groups, such as pyridyl, halogen atoms, such as chlorine or bromine, nitrile, hydroxy, alkoxy and alkoxycarbonyl groups. Preferably, the groups

represented by $R^1$ and $R^2$ each have from 1 up to 30 carbon atoms, more preferably up to 20 carbon atoms. $R^1$ is preferably a $C_1$ to $C_{20}$ alkenyl group, especially omega alkenyl. Preferred groups represented by $R^1$ are 1-decenyl, prenyl, but-2-enyl and hept-5-enyl, with 1-decenyl being especially preferred. $R^2$ preferably represents a lower alkyl group having from 1 to 10, more preferably from 1 to 5 carbon atoms. $R^2$ is preferably methyl.

Hal is preferably C1 or Br. The alkali metal M is preferably Na or K. The phase-transfer catalyst (hereinafter "PTC") may be any suitable PTC, for example, a quaternary onium compound of the formula $(R_1R_2R_3R_4X)Y$, in which X represents N, P or As, $R_1$, $R_2$, $R_3$ and $R_4$ each represent an alkyl, aralkyl, alkaryl or aryl group optionally substituted by one or more substituents, and Y represents a monovalent ion, e.g. a halide such as a chloride, bromide or iodide, or an hydrogen- or alkylsulphate group, a sulphonium or sulphoxonium group, or macrocyclic polyethers known as "crown ethers". Suitable onium compounds are tetraalkyl ammonium comounds, trialkylarylammonium compounds and alkyltriarylphosphonium comounds. The onium compound may be employed as the functional portion of a resin, e.g. an anion exchange resin. Preferred is tetrabutylammonium bromide.

The halide of formula $R^1$Hal may be prepared by reacting the corresponding dialkene with a hydrohalogenating agent. Thus, a suitable starting material is a diene such as 1,9-decadiene (hereinafter "1,9-DCD") which is readily available.

If desired, the ester can be hydrolysed to give the corresponding alcohol $R^1$OH, by generally known procedures. For example, by reacting with aqueous NaOH.

The invention is particularly suitable for the preparation of DAC from BDE. DAC bay be converted to DOL. BDE may be prepared from 1,9-DCD via anti-Markownikov hydrobromination.

As an alternative example of the invention, prenyl chloride may be converted to prenyl acetate.

The following Examples illustrate the invention, and also processes conducted for the purpose of comparison.

### Example 1

### Preparation of BDE from 1,9-DCD

1,9-DCD (1 molar equivalent) is charged to a reactor. The system is cooled to $0°$C, in the presence of a radical initiator (tertiary-butylhydroperoxide or azo-iso-butyronitrile) or under the influence of UV light. Hydrobromination takes place by the introduction of HBr gas via a dip-pipe under stirring. Approx. 0.5 molar equivalents of HBr are introduced at such a rate that the temperature remains $0°$C. After the addition of HBr (approx. 45% conversion of 1,9-DCD) the reactor contents are stripped with nitrogen (or washed with caustic, followed by phase separation) and the resulting mixture is distilled to obtain pure 10-bromo-1-decene.

### Example 2

### Preparation of DAC from BDE

Bromodecene (as commercially-available or from Example 1; 1 molar equivalent) plus approx. 1.1x its own weight of xylene, potassium acetate (1.2 molar equivalents) and TBAB (tetrabutylammonium bromide, catalytic amount, ca. 5 wt% on BDE) are charged to a reactor and the mixture is stirred. The system is closed and heated to $100°$C. Stirring is continued for 5 hours. The mixture is cooled to ambient temperature and, after the addition of water (approx. 1.2 w/w on bromodecene), the phases are allowed to separate. The lower aqueous layer containing KBr and unreacted KOAc is run off. The solvent is vacuum flash-evaporated resulting in crude DAC.

The dry xylene fraction can be recycled in the process. Care should be taken to allow the reaction to proceed for long enough to arrive at complete conversion of 10-bromodecene, since product purification may be hampered by the presence of this starting material (GLC analysis).

### Example 3

### Preparaton of DOL from DAC

The stirred mixture is cooled to $60°$C and 20% aq. NaOH (1.2 molar equivalents on bromodecene) is charged. The mixture is kept at $60°$C under stirring for 3 hours, cooled to ambient, and the two phases are allowed to separate. The lower, aqueous (NaOAc) phase is run off to disposal. The organic layer (omega-decencl) is batch-distilled (99° C, 4 mbar) to obtain pure omega-decenol.

Hydrolysis may be conducted using KOH instead of NaOH; the KOAc may be dried and recycled.

To overcome the presence of nitrogen-containing contaminants (from TBAB, e.g. tributylamine) in the final product, an extra water wash/phase separation before the product distillation may be needed.

Examples 4 to 15

Preparation of DAC from BDE

Various procedures have been run. Examples 4 to 9 are comparative. The results are given in the Table below.

In Examples 4 and 5, BDE was acetoxylated in acetic anhydride and acetic acid, with Aliquat 336 (Trade Mark) (tricaprylmethylammonium chloride) as catalyst. The procedure of Examples 4 and 5 was repeated in Examples 6 to 9, with the exception that acetic anhydride was omitted from the reaction mixture. First, BDE was converted to DAC at 128° C at atmospheric pressure (Ex. 4). The conversion of BDE to DAC, however, was incomplete after 22 hours. At 140° C the conversion was almost complete in 8 hours (Ex. 5).

DAC was isolated by removal of the NaBr precipitate and acetic acid by filtration and flash-distillation respectively. It appeared, however, that most of the applied excess of NaOAc remained in solution after removal of NaBr by filtration. After flash-distillation of the acetic acid, however, the DAC-containing residue solidified, caused by precipitation of the NaOAc. Although the precipitate could readily be dissolved in water, the solidification is highly undesired on large scale. It could be prevented by using a 10% instead of a molar excess of NaOAc, but then the reaction was incomplete at 140° C (Ex. 7). Excellent results were obtained at 120° C, but at the cost of a long reaction time (Ex. 8). Moderate results were obtained when NaOAc was replaced by KOAc (Ex. 9). From these results, it can be concluded that acetic acid is not applicable for a large-scale process.

In Examples 10 to 12, the reaction was conducted in toluene solution with 3 mol % tetrabutylammonium bromide (TBAB) as PTC. Poor results were obtained with NaOAc as reagent at 112° C (Ex. 10), whereas reaction with KOAc at 100° C resulted in an unexpected high conversion of BDE and selectivity to DAC (Ex. 11). It should be noted that in the latter experiment a jelly-like KBr precipitate was formed that might have deactivated the KOAc and/or PTC. Increasing the reaction temperature to 140° C gave rise to up to 99% conversion and selectivity (Ex. 12 to 14), despite the fact that TBAB decomposed to, inter alia, tributylamine at the reaction temperature.

In Examples 13 to 15, the reaction was conducted in xylene solution, with 3 mol % TBAB as catalyst, at atmospheric pressure. Good results were obtained with both NaOAc and KOAc (Ex. 13 and Ex. 14 respectively). TBAB was found to be stable at 100° C. At this temperature, the reaction was complete in 4.5 hours with a selectivity to DAC of almost 100%. Work-up of the reaction mixture was as follows: solid KBr and the excess of KOAc were dissolved in water, the aqueous phase was separated and xylene removed by flash distillation, leaving almost pure DAC.

In the following Table, the conversion and selectivity values are recorded as %GC area.

4

TABLE

| Example | Time h | Temp. °C | Pressure bar g | Solvent | Solvent/BDE v/v | M | Mol ratio BDE/HOAc | Mol ratio BDE/Ac₂O | Conv. BDE | Sel. DAC | Sel. DOL |
|---|---|---|---|---|---|---|---|---|---|---|---|
| 4 | 22 | 128 | 0 | HOAc | 2.5/1 | Na | 1/2 | 1/.7 | 94 | 72 | 1.4 |
| 5 | 8 | 140 | 1.5 | HOAc | 2/1 | Na | 1/2 | 1/.7 | 99 | 97 | 3 |
| 6 | 8 | 140 | 2.2 | HOAc | 3/1 | Na | 1/2 | - | 98 | 99 | 0.7 |
| 7 | 8 | 140 | 2.2 | HOAc | 2/1 | Na | 1/1.1 | - | 76 | 97 | 1.3 |
| 8 | 20 | 120 | 0 | HOAc | 3/1 | Na | 1/1.1 | - | 98 | 86 | 7 |
| 9 | 20 | 120 | 0 | HOAc | 3/1 | K | 1/1.1 | - | 84 | 99 | 0 |
| 10 | 21 | 112 | 0 | Toluene | 3/1 | Na | 1/1.1 | - | 18 | 89 | 2 |
| 11 | 4 | 100 | 0 | Toluene | 1.7/1 | K | 1/1.2 | - | 91 | 99 | 0 |
| 12 | 4 | 140 | 2.2 | Toluene | 4/1 | K | 1/1.2 | - | 99 | 99 | 0 |
| 13 | 8 | 140 | - | Xylene | 1.7/1 | Na | 1/1.1 | - | 90 | 99 | - |
| 14 | 6.5 | 140 | - | Xylene | 1.7/1 | K | 1/1.1 | - | 96 | 99 | - |
| 15 | 4.5 | 100 | - | Xylene | 1.7/1 | K | 1/1.2 | - | 100 | 99 | - |

Example 16

Preparation of prenol (3-methyl-2-buten-1-ol)

a) Isoprene (100g, 1.47mol) was added to a reactor vessel charged with 35%wt aqueous hydrochloric acid and the resulting mixture allowed to react for 6 hours at a temperature of from 0 to 5°C, with stirring. The two phases of the resulting mixture were allowed to separate and the phase containing prenyl chloride (4-chloro-2-methyl-2-butene) was drawn off and dried using sodium sulphate.

b) The prenyl chloride product of a) (containing 52.2g, 0.5mol of prenyl chloride), sodium acetate (45.4g, 0.55mol), toluene (50g, 0.54mol) and tetrabutylammonium bromide (3.24g, 0.01mol) were added to a reactor vessel. The resulting mixture was heated to 110°C and allowed to react for 3 hours with stirring. After this time, the resulting mixture was cooled and water was added to dissolve the precipitate of sodium chloride and any urneacted sodium acetate. The aqueous phase was drawn off, after which the toluene solvent was removed by flash-evaporation to yield prenyl acetate (4-acetoxy-2-methyl-2-butene).

c) The prenyl acetate product of b) (64.5g, 0.5mol), 15% wt aqeuous sodium hydroxide (148g, 0.55mol) and tetrabutylammonium bromide (4.9g, 0.015mol) were added to a reactor vessel, heated to 60°C and allowed to react for 1 hour with stirring. After this time, the resulting mixture was cooled and the aqueous phase drawn off. Prenol (3-methyl-2-buten-1-ol) was obtained from the remaining phase by distillation in a yield of 30.0g(70%).

## Claims

1. A process for preparing an ester of the formula

   $R^1$-O-CO-$R^2$

   wherein $R^1$ is an alkenyl group and $R^2$ in an alkyl, alkenyl or alkynyl group, which comprises reacting the corresponding halide of formula $R^1$Hal with the corresponding alkali metal acylate of the formula $MOCOR^2$, in the presence of an inert aprotic solvent and a phase-transfer catalyst.

2. A process according to claim 1, which additionally comprises adding water or another solvent for MHal, and separating the resultant solution of MHal.

3. A process according to claim 1 or claim 2, which comprises removing the inert apolar solvent from the ester.

4. A process according to any preceding claim, wherein Hal is Cl or Br and the alkali metal M is Na or K.

5. A process according to any preceding claim, wherein $R^2$ is methyl.

6. A process according to any preceding claim, wherein the catalyst is a tetraalkylammonium compound.

7. A process according to any preceding claim, wherein the solvent is toluene or xylene.

8. A process according to any preceding claim, which additionally comprises preparing the halide by reacting the corresponding alkene with a hydrohalogenating agent.

9. A process according to claim 10, wherein $R^1$ is dec-9-enyl.

10. A process according to any preceding claim, which additionally comprises hydrolysing the ester, to prepare the corresponding alcohol of formula $R^1$OH.

## DOCUMENTS CONSIDERED TO BE RELEVANT

EP 91200380.3

| Category | Citation of document with indication, where appropriate, of relevant passages | Relevant to claim | CLASSIFICATION OF THE APPLICATION (Int. Cl.5) |
|---|---|---|---|
| A | <u>GB - A - 917 568</u> (THE DISTILLERS) * Totality * | 1,4,6, 8 | C 07 C 69/145 C 07 C 67/11 C 07 C 33/025 C 07 C 27/02// C 07 C 21/14 |
| A | <u>GB - A - 1 480 636</u> (CHEM. WERKE HÜLS) * Claims; examples * | 1,2,4, 5 | |
| D,A | <u>US - A - 4 297 506</u> (K.D. COWAN) * Totality * | 1,3,4, 8-10 | |
| A | <u>US - A - 4 247 723</u> (A.J. DE JONG) * Totality * | 1,10 | |
| D,A | PATENT ABSTRACTS OF JAPAN, unexamined applications, C section, vol. 1, No. 12, March 22, 1977 THE PATENT OFFICE JAPANESE GOVERNMENT page 570 C 76 * Kokai-No. 51-125 204 (MITSUBISHI YUKA KK) * | 8,10 | TECHNICAL FIELDS SEARCHED (Int. Cl.5) C 07 C 69/00 C 07 C 67/00 C 07 C 33/00 C 07 C 27/00 C 07 C 21/00 |

The present search report has been drawn up for all claims

| Place of search | Date of completion of the search | Examiner |
|---|---|---|
| VIENNA | 12-06-1991 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)